(19) Europäisches Patentamt
European Patent Office
Office européen des brevets

(11) **EP 4 451 810 A1**

(12) **EUROPEAN PATENT APPLICATION**
published in accordance with Art. 153(4) EPC

(43) Date of publication:
**23.10.2024   Bulletin 2024/43**

(21) Application number: **22906919.0**

(22) Date of filing: **12.08.2022**

(51) International Patent Classification (IPC):
**H05H 13/02** (2006.01)   **A61N 5/10** (2006.01)

(52) Cooperative Patent Classification (CPC):
**A61N 5/10; H05H 13/02**

(86) International application number:
**PCT/JP2022/030802**

(87) International publication number:
**WO 2023/112383 (22.06.2023 Gazette 2023/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:   **13.12.2021   JP 2021202064**

(71) Applicant: **Hitachi High-Tech Corporation**
**Minato-ku**
**Tokyo 105-6409 (JP)**

(72) Inventor: **HAE, Takamitsu**
**Tokyo 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(54) **ACCELERATOR, PARTICLE BEAM THERAPY SYSTEM, AND CONTROL METHOD**

(57)   Provided is an accelerator that can accurately extract a charged particle beam having desired energy. An acceleration voltage measurement circuit 10 outputs an acceleration voltage signal $V_{acc}$ as a measurement signal in which an acceleration radiofrequency voltage excited in the accelerating cavity 9 is measured. The low power radiofrequency control system 25 inputs, to the accelerating cavity 9, as radiofrequency power, control radiofrequency power at a constant frequency included in the range of the resonance frequency of the accelerating cavity 9. When the phase difference between the acceleration voltage signal $V_{acc}$ and the control radiofrequency power becomes within the predetermined value, the low power radiofrequency control system 25 inputs, to the accelerating cavity 9, as radiofrequency power by feedback control, the acceleration radiofrequency power synchronized with the acceleration frequency that is the frequency of the acceleration voltage signal $V_{acc}$, and thereafter, when the acceleration frequency reaches an output frequency $F_{ext}$, stops the input of the acceleration radiofrequency power to the accelerating cavity 9.

*FIG. 5*

**Description**

Technical Field

The present disclosure relates to an accelerator, a particle therapy system, and a control method.

Background Art

As an accelerator that accelerates and extracts a charged particle beam, a circular accelerator that accelerates a charged particle beam by applying an acceleration electric field whose frequency is temporally modulated in a main magnetic field whose intensity is temporally constant has attracted attention. Since this type of circular accelerator can generate a main magnetic field using a superconducting coil, it is advantageous for reduction in size and cost, and is applied in particular to a particle therapy system and the like.

In a circular accelerator, a tuning cavity may be used as an accelerating cavity that excites an acceleration radiofrequency electric field for accelerating a charged particle beam. In this case, the resonance frequency of the accelerating cavity is temporally modulated by temporally changing the capacitance or inductance of the accelerating cavity. At this time, the radiofrequency power input to the accelerating cavity needs to be tuned with the accelerating cavity (to have substantially the same frequency as the resonance frequency of the accelerating cavity). Techniques of modulating the resonance frequency of an accelerating cavity include a technique of installing the accelerating cavity with a rotating capacitor having a pair of rotor and stator electrodes facing each other as a modulator that modulates the accelerating cavity (see PTL 1).

Examples of the circular accelerator include a synchrocyclotron and an eccentric orbital accelerator described in PTL 1. In the synchrocyclotron, since the extraction energy, which is the energy of the charged particle beam to be extracted, is fixed, the radiofrequency power input to the accelerating cavity after a certain period of time from when the beam is injected is cut off. On the other hand, in the eccentric orbital accelerator described in PTL 2, since the extraction energy is variable, the radiofrequency power input to the accelerating cavity needs to be cut off at an appropriate timing according to the extraction energy.

Citation List

Patent Literature

PTL 1: US 2010/0045213
PTL 2: JP 2019-133745 A

Summary of Invention

Technical Problem

In an accelerator using a tuning cavity as an accelerating cavity, radiofrequency power input to the accelerating cavity needs to be tuned with the accelerating cavity, but the modulation pattern of the accelerating cavity has an error depending on manufacturing tolerance and control variation of the modulator, temperature of the accelerating cavity, and the like.

In the technique described in PTL 2, since, as acceleration radiofrequency power, a programmed frequency pattern signal is input to the accelerating cavity via the radiofrequency power amplifier, it is strongly affected by an error of the modulation pattern, and an error occurs in the energy of the charged particle beam. Since the method described in PTL 1 is for a synchrocyclotron, it is not possible to cut off the acceleration radiofrequency power at an appropriate timing according to the desired extraction energy particularly when the method is applied to an eccentric orbital accelerator.

An object of the present invention is to provide an accelerator, a particle therapy system, and a control method that can accurately extract a charged particle beam having desired energy.

Solution to Problem

An accelerator according to one aspect of the present disclosure is an accelerator that accelerates a charged particle beam while causing the charged particle beam to orbit by a temporally constant main magnetic field and an acceleration radiofrequency voltage, the accelerator includes: an accelerating cavity that excites the acceleration radiofrequency voltage according to a time-varying resonance frequency by using radiofrequency power having been input; a measurement unit that outputs a measurement signal obtained by measuring the acceleration radiofrequency voltage; and a control unit that inputs, as the radiofrequency power, control radiofrequency power having a constant frequency included in a range of the resonance frequency to the accelerating cavity, inputs, as the radiofrequency power, acceleration radiofrequency power synchronized with an acceleration frequency that is a frequency of the measurement signal by feedback control to the accelerating cavity when a phase difference between the measurement signal and the control radiofrequency power falls within a predetermined value, and stops input of the acceleration radiofrequency power to the accelerating cavity when the acceleration frequency reaches a required value.

Advantageous Effects of Invention

According to the present invention, it becomes possible to accurately extract a charged particle beam having desired energy.

Brief Description of Drawings

[FIG. 1] FIG. 1 is a perspective view illustrating an appearance example of a circular accelerator according to an embodiment of the present disclosure.
[FIG. 2] FIG. 2 is a transverse cross-sectional view illustrating a configuration example of a transverse cross section of a circular accelerator according to an embodiment of the present disclosure.
[FIG. 3] FIG. 3 is a longitudinal cross-sectional view illustrating a configuration example of a longitudinal cross section of a circular accelerator according to an embodiment of the present disclosure.
[FIG. 4] FIG. 4 is a view illustrating an example of an orbit of a charged particle beam for each energy.
[FIG. 5] FIG. 5 is a view illustrating a configuration example of a low power radiofrequency control system.
[FIG. 6] FIG. 6 is a timing chart for explaining an example of a control sequence of the low power radiofrequency control system.
[FIG. 7] FIG. 7 is a view illustrating a configuration example of a particle therapy system according to an embodiment of the present disclosure.

Description of Embodiments

Hereinafter, an embodiment of the present disclosure will be described with reference to the drawings.

First Embodiment

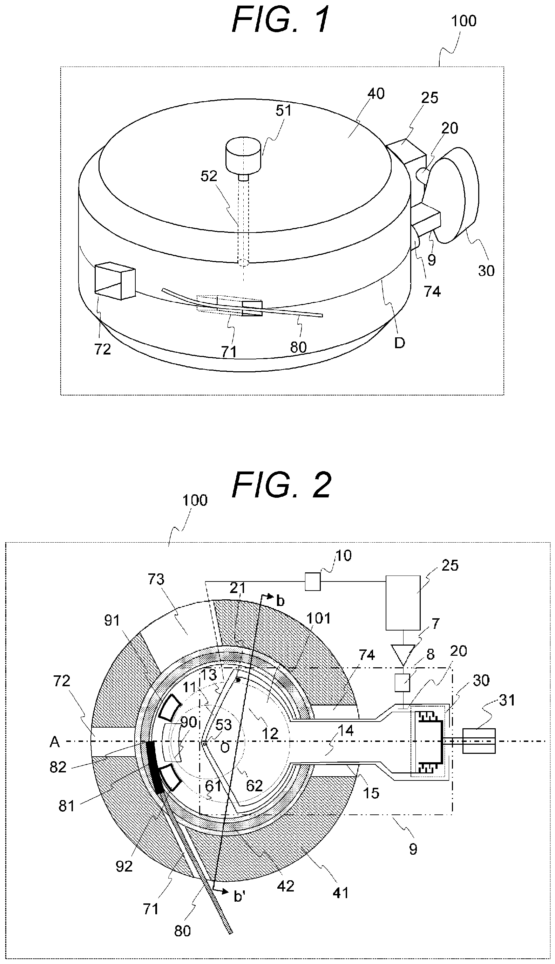

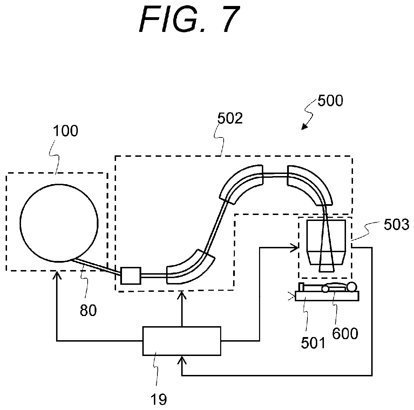

FIGS. 1 to 3 are views illustrating an example of a circular accelerator according to an embodiment of the present disclosure. An accelerator 100 illustrated in FIGS. 1 to 3 is a circular accelerator that accelerates, while orbiting, a charged particle beam (hereinafter, may be simply called a beam) by applying an acceleration radiofrequency voltage having been subjected to frequency modulation in a main magnetic field having a temporally constant intensity. In the present embodiment, the accelerator 100 accelerates a proton beam using protons as charged particles to an arbitrary energy of 70 MeV to 235 MeV and extracts the proton beam. However, the charged particle beam may be a baryon beam using helium, carbon, or the like, and the extraction energy, which is the energy of the beam to be extracted, is not limited to the range of 70 MeV to 235 Mev. The accelerator 100 is suitable for a particle therapy system (see FIG. 7), but is not limited to the application.

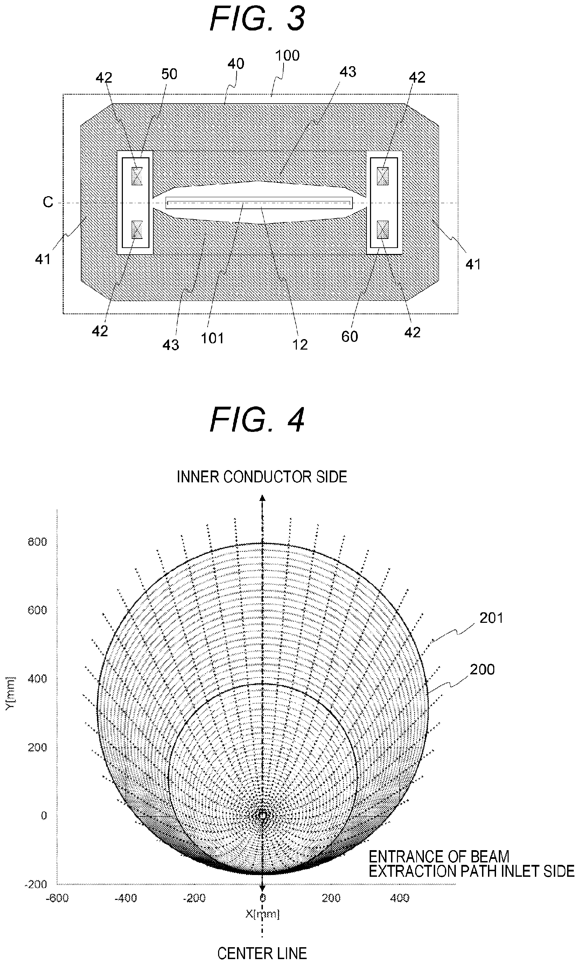

FIG. 1 is a perspective view illustrating the appearance of the accelerator 100, FIG. 2 is a transverse cross-sectional view illustrating the configuration of a transverse cross section obtained by cutting the accelerator 100 along a center plane in a longitudinal direction, and FIG. 3 is a longitudinal cross-sectional view illustrating the configuration of a longitudinal cross section obtained by cutting the accelerator 100 along b-b' line in FIG. 2.

The accelerator 100 includes a main electromagnet 40 that can be divided into an upper part and a lower part across a center plane D, and an acceleration region 101, which is a substantially cylindrical space for accelerating a beam, is formed inside the main electromagnet 40. The acceleration region 101 is formed substantially vertically symmetrically with respect to the center plane D, and is vacuumed.

The main electromagnet 40 includes a yoke 41, a main coil 42, and a main magnetic pole 43. The yoke 41 forms an outer shell of the main electromagnet 40 and forms the acceleration region 101 inside. The main coil 42 is an annular superconducting coil, and is installed in each of the up-down direction across the center plane D. Each of the main coils 42 is installed along the inner wall of the yoke 41. A cryostat 50 that is a cooling mechanism for cooling the main coil 42 to a certain temperature (temperature at which the main coil 42 exhibits complete diamagnetism) or less is installed around the main coil 42. The main magnetic pole 43 is installed on the inner peripheral side of the main coil 42 in each of the up-down direction across the center plane D.

When the main coil 42 is supplied with a current, a temporally constant magnetic field called a main magnetic field is excited in the acceleration region 101 by the main magnetic pole 43, and the beam orbits on an orbital plane of the acceleration region 101 due to the influence of the main magnetic field. At this time, the beam orbits a different orbit for each energy. Hereinafter, each orbit for each energy where the beam orbits is called an orbit. In the present embodiment, the main electromagnet 40 is formed such that the center of the orbit of the beam is eccentric (deviated from the physical center of the acceleration region 101) (see FIG. 4). That is, the accelerator 100 of the present embodiment is an eccentric orbital accelerator. FIG. 2 illustrates, among the orbits, a maximum energy orbit 61 where a beam having the maximum energy orbits and a minimum extraction energy orbit 62 having the energy of 70 Mev.

An ion source 51 that generates a beam injected into the acceleration region 101 present inside the main electromagnet 40 is installed in an upper part of the main electromagnet 40. The ion source 51 is, for example, an electron cyclotron resonance (ECR) ion source or the like. The ion source 51 is connected to the ion injection portion 53 in the acceleration region 101 via a low energy beam transport system 52. Due to this, the beam generated by the ion source 51 is injected into the acceleration region 101 from the ion injection portion 53 via the low energy beam transport system 52. In the present embodiment, the ion injection portion 53 is disposed on a side of an entrance of the beam extraction path 82, which is an injection port of a magnetic channel 81 for extracting a beam to the outside, relative to a physical center O of the acceleration region 101 on a center line A passing through the center of the accelerator 100. The ion source 51 may be disposed inside the acceleration region 101. In this case, a Penning ionization gauge (PIG) type ion source or the like is suitable as the ion source 51.

As illustrated in FIGS. 1 and 2, the yoke 41 is provided with a plurality of through holes. For example, the yoke 41 is provided with a beam through hole 71, a coil through hole 72, a vacuum through hole 73, and a radiofrequency system through hole 74 as through holes on the center plane D of the main electromagnet 40.

The beam through hole 71 is a through hole for extracting a beam, and is provided with a high energy beam transport system 80 for extracting a beam from the inside to the outside of the yoke 41. The high energy beam transport system 80 is connected with the magnetic channel 81. The coil through hole 72 is a through hole for drawing various coils (such as the main coil 42) provided inside the yoke 41 to the outside. The vacuum through hole 73 is a through hole for vacuuming the acceleration region 101. The radiofrequency system through hole 74 is a through hole for inserting an accelerating cavity 9.

The accelerating cavity 9 is a member that excites an acceleration radiofrequency voltage for accelerating the beam injected into the acceleration region 101 from the ion injection portion 53. In the present embodiment, the accelerating cavity 9 is a λ/2 resonance type cavity, and includes a dee electrode 12, a dummy dee electrode 13, an inner conductor 14, an outer conductor 15, and a rotating capacitor 30.

The dee electrode 12 is a hollow electrode through which the beam passes inside, and has a substantially fan shape having a predetermined divergence angle with a point in the vicinity of the ion injection portion 53 as a vertex. The inner conductor 14 is a conductor connected to the dee electrode 12 and extending from the dee electrode 12 to the outside of the main electromagnet 40. The outer conductor 15 is a conductor that externally covers the dee electrode 12 and the inner conductor 14. The dummy dee electrode 13 is an electrode having a ground potential and is connected to the outer conductor 15. The dummy dee electrode 13 is provided so as to face the dee electrode 12, and an acceleration gap 11 in which an acceleration radiofrequency voltage is excited is formed between the dee electrode 12 and the dummy dee electrode 13.

The rotating capacitor 30 is a modulator for modulating the resonance frequency of the accelerating cavity 9. Specifically, the rotating capacitor 30 temporally changes the resonance frequency of the accelerating cavity 9 by changing to capacitance according to the rotation angle of the rotation electrode (not illustrated) rotated by a motor 31. The accelerating cavity 9 excites, in the acceleration gap 11, an acceleration radiofrequency voltage corresponding to this time-varying resonance frequency. The rotating capacitor 30 or the motor 31 is provided with a detection unit (not illustrated) that detects and outputs, as an angle signal, the rotation angle of the rotation electrode. The acceleration gap 11 is formed according to the beam orbit shape. In the present embodiment, the number of harmonics is 1, that is, the orbit frequency at which the beam orbits and the acceleration frequency, which is the frequency of the acceleration radiofrequency voltage (more specifically, the frequency of the acceleration electric field by the acceleration radiofrequency voltage) are substantially the same.

The accelerating cavity 9 is connected to a low power radiofrequency control system 25 via an input coupler 20, and a radiofrequency power amplifier 7 and an input radiofrequency measurement circuit 8 are provided between the input coupler 20 and the low power radiofrequency control system 25.

The low power radiofrequency control system 25 outputs low power radiofrequency power. The radiofrequency power amplifier 7 amplifies and inputs, to the accelerating cavity 9 via the input coupler 20, the radiofrequency power output from the low power radiofrequency control system 25. The input coupler 20 is a device for inputting radiofrequency power to the accelerating cavity, and is connected to the inner conductor 14 of the accelerating cavity 9 by electrostatic coupling or magnetic coupling. The accelerating cavity 9 excites an acceleration radiofrequency voltage in the acceleration gap 11 by radiofrequency power input via the input coupler 20, and generates an acceleration electric field that accelerates a beam by the acceleration radiofrequency voltage. The input radiofrequency measurement circuit 8 measures radiofrequency power input to the accelerating cavity 9 as described later.

In the accelerator 100 described above, the charged particle beam generated by the ion source 51 is injected from the ion injection portion 53 via the low energy beam transport system 52 into the acceleration region 101 formed inside the main electromagnet 40. The injected beam is accelerated by the acceleration electric field excited in the accelerating cavity 9, and orbits in the main magnetic field while increasing the energy. With an increase in the energy of the beam, the radius of curvature of the beam orbit increases, and therefore the beam draws a helical orbit.

In the present embodiment, the orbit that is an orbit of the beam for each energy becomes an eccentric orbit. Hereinafter, the main magnetic field that achieves the eccentric orbit will be described.

The main magnetic field may be a magnetic field whose intensity is constant with respect to the circumferential direction of the orbit, or may be an azimuthal varying field (AVF) type magnetic field whose intensity changes in the circumferential direction. In any case, the main magnetic field is an anisochronous magnetic field and is generated to satisfy a beam stability condition. The beam stability condition is that the n value expressed by the following Expression (1) becomes greater than 0 and less than 1.

[Expression 1]

$$ n = -\frac{\rho}{|B|}\frac{\partial B}{\partial r} \qquad \cdots (1) $$

Here, $\rho$ is the deflection radius of the orbit, B is the intensity of the main magnetic field, and $\partial B/\partial r$ is the magnetic field gradient in the radial direction. The r axis is an axis that faces outward in the radial direction when the orbital plane is expressed in a two-dimensional polar coordinate system with the center of the acceleration region 101 as the origin.

When the beam stability condition is satisfied, a beam slightly shifted in the radial direction from the orbit receives a restoring force from the main magnetic field in a direction of returning the beam to the orbit, and a beam shifted in a direction vertical with respect to the orbital plane receives a restoring force from the main magnetic field in a direction of returning the beam to the orbital plane. That is, the beam stably orbits and accelerates while performing betatron oscillation in the vicinity of the orbit. In the beams of all energies, the betatron frequency (horizontal tune) $v_r$ in the direction parallel to the orbital plane and orthogonal to the orbit has a value close to 1. The main magnetic field is formed by the main magnetic pole 43 and a trim coil and a magnetic pole piece (both not illustrated) installed on the surface of the main magnetic pole 43, and these constituent elements are arranged vertically symmetrically with respect to the orbital plane. Therefore, the main magnetic field has only a component in a direction perpendicular to the orbital plane on the orbital plane.

FIG. 4 is a view illustrating an orbit of a beam that orbits in the main magnetic field for each energy. In FIG. 4, the vertical axis is an axis facing the inner conductor 14 of the accelerating cavity 9 from the entrance of the beam extraction path 82, and the origin 0 is the position of the ion injection portion 53. FIG. 4 illustrates, as an orbit 200, an orbit of the beam having the maximum energy of 235 MeV positioned on the outermost side and 50 types of orbits in which the magnetic rigidity modulus of the beam is different by 0.04 Tm from the orbit.

As illustrated in FIG. 4, in the eccentric orbital accelerator 100, in a case where the energy of the beam is low, the orbit 200 is approximated to a concentric orbit centered on the vicinity of the ion injection portion 53 similarly to a cyclotron accelerator or the like. However, in a case where the energy of the beam is large, each orbit 200 does not become a concentric orbit, and is aggregated on the entrance of the beam extraction path 82 side and becomes a discrete orbit on the inner conductor 14 side. Hereinafter, a region where the orbits 200 are aggregated may be called an aggregation region, and a region where the orbits 200 are discrete may be called a discrete region.

In FIG. 4, an equal orbit phase line 201 connecting the same orbit phase of each orbit 200 is illustrated for each orbit phase $\pi/20$ from an aggregation region 203. The acceleration gap 11 formed between the dee electrode 12 and the dummy dee electrode 13 is provided along any of the equal orbit phase line 201 (e.g., the equal orbit phase line 201 orbiting ±90 degrees as viewed from the aggregation point where the orbits 200 are closest to each other). More specifically, the dee electrode 12 has a hollow shape such as a fan shape with a tip end near the center of the orbit 200 and with a radius along the equal orbit phase line 201.

The description returns to FIGS. 1 to 3. In the main electromagnet 40, in order to extract the orbiting beam to the outside, a radiofrequency kicker 90, and a peeler magnetic field region 91 and a regenerator magnetic field region 92, which are disturbance magnetic fields composed of a two-pole magnetic field or a multipole magnetic field, are formed. The radiofrequency kicker 90 is installed in the beam aggregation region, and the peeler magnetic field region 91 and the regenerator magnetic field region 92 are installed on the outer peripheral side relative to the maximum energy orbit 61.

The radiofrequency kicker 90 applies the beam with an extraction radiofrequency voltage for extracting the beam to the outside. The frequency of the extraction radiofrequency voltage is set to be substantially the same as the product of the fractional part of the horizontal tune $v_r$ of the beam having the desired extraction energy and the orbit frequency of the beam having the desired extraction energy. The extraction radiofrequency voltage may be a radiofrequency voltage having a finite frequency bandwidth including a frequency component substantially the same as this product.

Due to the extraction radiofrequency voltage from the radiofrequency kicker 90, the amplitude of the horizontal betatron oscillation of the beam continues to increase resonantly, and the beam reaches the peeler magnetic field region 91 and the regenerator magnetic field region 92. A second order resonance occurs in the peeler magnetic field region 91 and the regenerator magnetic field region 92, whereby the beam is kicked outside the maximum energy orbit 61 and reaches the entrance of the beam extraction path 82. After that, the beam is guided into the magnetic channel 81 and is sufficiently deflected to be extracted to outside the accelerator 100 through the high energy beam transport system 80.

Thus, in the present embodiment, since the beam is extracted from the vicinity of the aggregation region, it becomes possible to reduce the kick amount required for extraction of the beam, and it becomes possible to easily extract the beam having desired extraction energy.

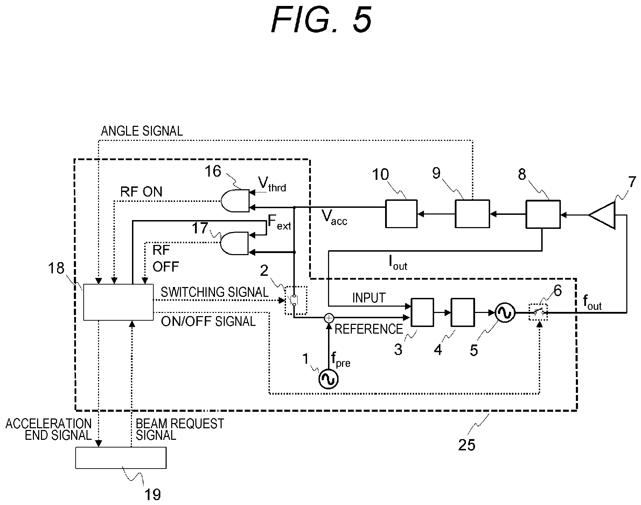

FIG. 5 is a view illustrating a configuration example of the low power radiofrequency control system 25. The low power radiofrequency control system 25 illustrated in FIG. 5 is a control unit for controlling the accelerating cavity 9, and includes an oscillator 1, a changeover switch 2, a time to digital converter (TDC) 3, a digital loop filter 4, a digital controlled oscillator (DCO) 5, a radio frequency (RF) switch 6, a voltage amplitude comparator 16, a frequency comparator 17, and a controller 18. The low power radiofrequency control system 25 is connected to the accelerating cavity 9 via the radiofrequency power amplifier 7 and the input radiofrequency measurement circuit 8, and is further connected to an acceleration voltage measurement circuit 10 and a host control system 19. At least a part of the function of the host control system 19 described below may be included in the low power radiofrequency control system 25 (particularly, the controller 18) .

The oscillator 1 generates a frequency signal for controlling a start timing for starting input (start-up) of the acceleration radiofrequency power to the accelerating cavity 9. A frequency $f_{pre}$ of the frequency signal is included in a frequency modulation range, which is the range of the resonance frequency of the accelerating cavity 9. In the present embodiment, the frequency $f_{pre}$ of the frequency signal is set to a value lower than an upper limit frequency $f_{max}$ of the frequency modulation range and larger than a predetermined frequency $f_{inj}$, which is an orbit frequency corresponding to the injection energy that is the energy of the beam injected into the acceleration region 101. The oscillator 1 preferably can generate a frequency signal whose frequency is temporally and accurately constant, and is, for example, a crystal oscillator or the like.

The changeover switch 2 is a changeover unit that switches on/off of feedback control by a phase locked loop (PLL) circuit described later.

The TDC 3, the digital loop filter 4, and the DCO 5 constitute a PLL circuit in which an output signal of the oscillator 1 or a signal obtained by adding an output signal of the oscillator 1 and an acceleration voltage signal $V_{acc}$ is a reference signal, and an input current signal $T_{out}$, which is an output signal of the input radiofrequency measurement circuit 8, is an input signal. After the changeover switch 2 is brought into an on state, the PLL circuit adjusts the phase of the input signal so that the phase difference between the input signal and the reference signal becomes zero, thereby matching the frequency of the input signal to the frequency of the reference signal and outputting the signal. In the example of FIG. 5, the PLL circuit is an all digital PLL circuit in which all circuits (the TDC 3, the digital loop filter 4, and the DCO 5) include digital circuits. However, the PLL circuit may be a PLL circuit in which an analog circuit is used for some circuits, or may be a PLL circuit in which all circuits include analog circuits. The PLL circuit may use a system that converts each of the input signal and the reference signal into an IQ signal, detects a phase difference between the both signals, and controls output by an IQ modulator (not illustrated) such that the phase difference becomes zero by proportional integral control.

The RF switch 6 switches between input and stop, to the accelerating cavity 9, of radiofrequency power that is an output signal of the PLL circuit.

The radiofrequency power amplifier 7 amplifies and inputs, to the accelerating cavity 9 as input radiofrequency power, the radiofrequency power output from the low power radiofrequency control system 25. The radiofrequency power amplifier 7 is, for example, a semiconductor amplifier.

The input radiofrequency measurement circuit 8 between the radiofrequency power amplifier 7 and the accelerating cavity 9 is an input measurement unit that measures, using a directional coupler, input radiofrequency power that is a traveling wave component toward the accelerating cavity 9, and outputs the input radiofrequency power as an input current signal $I_{out}$. The signal output from the input radiofrequency measurement circuit 8 may be an input voltage signal in place of an input current signal. In FIG. 5 does not illustrate the input coupler 20 illustrated in FIGS. 1 and 2.

The acceleration voltage measurement circuit 10 is a measurement unit that measures the acceleration radiofrequency voltage applied to the acceleration gap 11 and outputs a measurement signal indicating the measurement value as the acceleration voltage signal $V_{acc}$. For example, the acceleration voltage measurement circuit 10 processes and outputs, as the acceleration voltage signal $V_{acc}$, a signal obtained from a pickup portion 21 near the acceleration gap 11.

The voltage amplitude comparator 16 compares an acceleration voltage value, which is the amplitude of the acceleration voltage signal $V_{acc}$, with a threshold value $V_{thrd}$, and when the acceleration voltage value reaches the threshold value $V_{thrd}$, outputs, to the controller 18, an RFON command signal instructing input of the acceleration radiofrequency power. When the above-described IQ modulator is used, the voltage amplitude comparator 16 can be omitted.

The frequency comparator 17 compares the acceleration frequency that is the frequency of the acceleration voltage signal $V_{acc}$ with an output frequency $F_{ext}$, which is the required value, and when the acceleration frequency reaches the output frequency $F_{ext}$, outputs, to the controller 18, an RFOFF command signal instructing stop of the acceleration radiofrequency power. The output frequency $F_{ext}$ is a value of an acceleration frequency corresponding to an orbit frequency at which a beam having desired extraction energy orbits the orbit.

By controlling the changeover switch 2 and the RF switch 6, the controller 18 controls start and stop of input of the acceleration radiofrequency power to the accelerating cavity 9.

The controller 18 holds a lookup table indicating a correspondence relationship between the energy of the beam and the orbit frequency in order to achieve the above control. Upon receiving, from the host control system 19, a beam request signal requesting beam extraction preparation, the controller 18 sets the output frequency $F_{ext}$ corresponding to desired extraction energy in the frequency comparator 17 according to the beam request signal. The beam request signal includes information indicating desired extraction energy.

Then, the controller 18 brings the changeover switch 2 into an off state, brings the RF switch 6 into an on state, and inputs a signal subjected to phase synchronization with the output signal of the oscillator 1 to the accelerating cavity 9 as control radiofrequency power. Thereafter, when the RFON signal is brought into a high state, the controller 18 brings the changeover switch 2 into an on state, and inputs, to the accelerating cavity 9 as the acceleration radiofrequency power, a signal in which the input current signal $I_{out}$ from the input radiofrequency measurement circuit 8 is subjected to phase synchronization with the acceleration voltage signal $V_{acc}$ from the acceleration voltage measurement circuit 10 using the PLL circuit. This maintains a state in which the acceleration radiofrequency power having a frequency substantially the same as the resonance frequency of the accelerating cavity 9 is input to the accelerating cavity 9, and enables the beam to be continuously accelerated.

The low power radiofrequency control system 25 illustrated in FIG. 5 is merely an example, and is not limited to the illustrated configuration. For example, the input measurement signal by the input radiofrequency measurement circuit 8 is not limited to the current signal, and may be a voltage signal. The measurement signal by the acceleration voltage measurement circuit 10 is not limited to a voltage signal and may be a current signal. A circuit for suppressing a phase shift of the output signal of the changeover switch 2 due to the switching of the node of the changeover switch 2 may be provided between the changeover switch 2 and the TDC 3 of the PLL circuit.

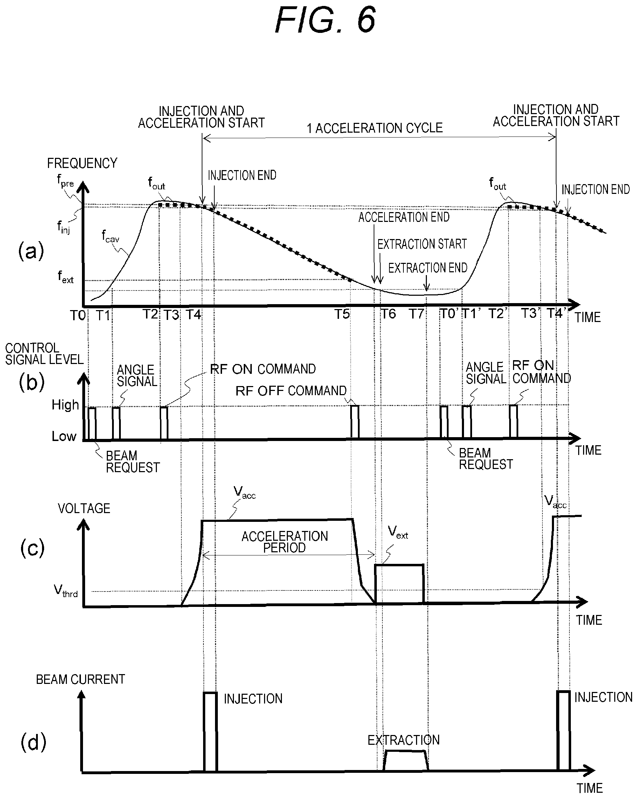

FIG. 6 is a timing chart for explaining a control sequence of the low power radiofrequency control system 25. FIG. 6(a) illustrates a temporal change of a resonance frequency $f_{cav}$ (solid line) of the accelerating cavity 9 and a frequency $f_{out}$ (broken line) of the input radiofrequency power, which is an output signal of the radiofrequency power amplifier 7. FIG. 6(b) illustrates each control signal used in the low power radiofrequency control system 25. FIG. 6(c) illustrates a temporal change of the acceleration voltage signal $V_{acc}$ by the acceleration voltage measurement circuit 10 and an extraction radiofrequency voltage $V_{ext}$ applied to the radiofrequency kicker 90. FIG. 6(d) illustrates a temporal change of the current of an injected beam and the current of an extracted beam.

The resonance frequency $f_{cav}$ of the accelerating cavity 9 changes in the same frequency modulation pattern for each acceleration cycle illustrated in FIG. 6 with the rotation of the rotating capacitor 30. Each control signal illustrated in FIG. 6 (b) is activated at a high level in the present embodiment, and then reset to a low level after a certain period of time.

First, upon receiving a beam request signal from the host control system 19 (time T0), the controller 18 starts the following processing. At this time, both the RF switch 6 and the changeover switch 2 are in an off state.

The controller 18 monitors the angle signal of the rotating capacitor 30, and, after the rotation angle of the rotating capacitor 30 becomes a predetermined angle (time T1), transmits an on signal to the RF switch 6 near a time point when the resonance frequency $f_{cav}$ turns to a monotonous decrease with respect to the time axis after a certain period of time elapses, and switches the RF switch 6 to the on state (time T2). The predetermined angle is an angle set only once in one acceleration cycle, and is set in advance to an angle at a timing when the resonance frequency $f_{cav}$

increases, for example.

Immediately after the RF switch 6 is brought into the on state, the resonance frequency $f_{cav}$ is higher than the frequency $f_{out}$ (= $f_{pre}$) of the output signal of the PLL circuit (the output signal of the DCO 5), and they are not in a tuning state. Thereafter, the resonance frequency $f_{cav}$ decreases with the rotation of the rotating capacitor 30 and becomes equal to the frequency $f_{out}$ (= $f_{pre}$) of the output signal of the PLL circuit, and the phase difference between the input signal of the TDC 3 and the reference signal approaches zero. Then, the frequency $f_{out}$ of the output signal of the PLL circuit (output signal of the DCO 5) is locked to the frequency $f_{pre}$ of the output signal of the oscillator 1, and the control radiofrequency power having the frequency $f_{out}$ substantially the same as the frequency $f_{pre}$ is output as the input radiofrequency power to the accelerating cavity 9 via the radiofrequency power amplifier 7.

While the difference between the resonance frequency $f_{cav}$ of the accelerating cavity 9 and the frequency $f_{out}$ of the input radiofrequency power is large (between times T2 and T3), the input radiofrequency power has a large component reflected by the accelerating cavity 9 and a small component input to the accelerating cavity 9. Thereafter, when the difference between the resonance frequency $f_{cav}$ of the accelerating cavity 9 and the frequency $f_{out}$ of the input radiofrequency power becomes small (approaches time T3), the reflection component of the input radiofrequency power sharply decreases, and the input radiofrequency power is input to the accelerating cavity 9. Due to this, the acceleration gap 11 is applied with the acceleration radiofrequency voltage, and the voltage amplitude of the acceleration voltage signal $V_{acc}$ becomes large.

Thereafter, when the amplitude of the acceleration voltage signal $V_{acc}$ reaches the threshold value $V_{thrd}$ at time T3, the RFON command signal is output from the voltage amplitude comparator 16 to the controller 18. Upon receiving the RFON command signal, the controller 18 transmits a switching signal to the changeover switch 2, brings the changeover switch 2 into an on state, and adds the acceleration voltage signal $V_{acc}$ to the reference signal of the PLL circuit (reference signal of the TDC 3) (time T3) . In the present embodiment, the fact that the amplitude of the acceleration voltage signal $V_{acc}$ reaches the threshold value $V_{thrd}$ corresponds to the fact that the phase difference between the acceleration voltage signal $V_{acc}$ and the control radiofrequency power becomes within a predetermined value. When the IQ modulator is used in the PLL circuit, the PLL circuit can detect that the phase difference between the acceleration voltage signal $V_{acc}$ and the control radiofrequency power becomes within the predetermined value, and thus the voltage amplitude comparator 16 can be omitted as described above.

Due to this, the frequency $f_{out}$ of the output signal of the PLL circuit is locked to the acceleration frequency of the acceleration voltage signal $V_{acc}$ substantially the same as the resonance frequency $f_{cav}$ of the accelerating cavity 9, and the acceleration radiofrequency power that changes following the time-varying resonance frequency $f_{cav}$ is input to the accelerating cavity 9 via the radiofrequency power amplifier 7 as the input radiofrequency power.

Thereafter, the controller 18 causes the beam to be injected into the acceleration region 101 in the main electromagnet 40 from the ion source 51 (time T4). The controller 18 controls the ion source 51 via the host control system 19, for example, to cause the beam to be injected.

Time T4 is a time when a certain period of time has elapsed from time T3. Time T4 may be a time when the frequency of the acceleration voltage signal $V_{acc}$ matches the predetermined frequency $f_{inj}$. The predetermined frequency $f_{inj}$ is an orbit frequency corresponding to injection energy that is the energy of the beam injected into the acceleration region 101. In this case, for example, a frequency comparator (not illustrated) is used to set such that a signal is output from this frequency comparator when the frequency of the acceleration voltage signal $V_{acc}$ matches the predetermined frequency $f_{inj}$, and when the controller 18 receives the signal, the beam is injected into the acceleration region 101. As illustrated in FIG. 6(c), an automatic gain controller (AGC) (not illustrated) that controls the amplitude of the radiofrequency power may be provided after the DCO 5 so that the amplitude of the acceleration voltage signal $V_{acc}$ becomes a set value before time T4.

Then, when the frequency of the acceleration voltage signal $V_{acc}$ reaches the output frequency $f_{ext}$, an RFOFF command signal is output from the frequency comparator 17 to the controller 18. Upon receiving the RFOFF command signal, the controller 18 transmits an OFF signal to the RF switch 6, switches the RF switch 6 to the OFF state, and stops the input of the acceleration radiofrequency power to the accelerating cavity 9 (time T5) . Due to this, the acceleration of the beam is stopped, and the beam continues to orbit an orbit in accordance with the energy.

The host control system 19 drives the radiofrequency kicker 90 to apply the extraction radiofrequency voltage $V_{ext}$ to the beam. Due to this, the beam reaches the peeler magnetic field region 91 and the regenerator magnetic field region 92, and is further kicked to the outside of the maximum energy orbit 61 by the peeler magnetic field region 91 and the regenerator magnetic field region 92 to reach the entrance of the beam extraction path 82. Thereafter, the beam is extracted to the outside of the accelerator 100 through the magnetic channel 81 and the high energy beam transport system 80 (time T6) . After the controller 18 sends an acceleration end signal to the host control system 19, the host control system 19 drives and stops the radiofrequency kicker 90 after performing various condition determinations.

Thereafter, when the extraction radiofrequency voltage $V_{ext}$ is stopped, the beam extraction is stopped (time T7) . The beam continues to be extracted while orbit charges, which are charged particles orbit as a beam, remain in the acceleration region 101. The beam is continuously or intermittently extracted when the host control system 19 adjusts the application time of the extraction radiofrequency voltage $V_{ext}$. When the beam cannot be detected by a beam dose monitor (not illustrated) disposed outside the accelerator even if the extraction radiofrequency voltage $V_{ext}$ is continuously applied, the host control system 19 may determine that there is no longer an orbiting charge.

When the beam is extracted again, the host control system 19 transmits the beam request signal to the controller 18 again (time T0').

FIG. 7 is a view illustrating a configuration example of a particle therapy system including the accelerator 100 described in FIGS. 1 to 6. In FIG. 7, a particle therapy system 500 includes the accelerator 100, a treatment couch 501, a beam transport device 502, an irradiation device 503, and the host control system 19.

The treatment couch 501 is a couch on which a patient 600, who is an irradiation target to be irradiated with the beam, is placed. The beam transport device 502 is connected to the high energy beam transport system 80 of the accelerator 100, and transports, to the irradiation device 503, the beam extracted from the accelerator 100.

The irradiation device 503 irradiates, as a target, the affected area of the patient 600 on the treatment couch 501 with the beam transported from the beam transport device 502. At this time, the irradiation device 503 irradiates, with the beam, each of a plurality of irradiation spots in which the affected area of the patient 600 is divided, measures the irradiation dose for each of the irradiation spots, and notifies the host control system 19 of the measured irradiation dose. The host control system 19 calculates a required dose for each of the irradiation spots, transmits a beam request signal to the controller 18, and controls the extraction radiofrequency voltage $V_{ext}$ so that the affected area is irradiated with a beam of a necessary dose.

As described above, according to the present embodiment, the acceleration voltage measurement circuit 10 outputs the acceleration voltage signal $V_{acc}$ as a measurement signal obtained by measuring the acceleration radiofrequency voltage excited in the accelerating cavity 9. The low power radiofrequency control system 25 inputs, to the accelerating cavity 9, as radiofrequency power, control radiofrequency power at a constant frequency included in the range of the resonance frequency of the accelerating cavity 9. When the phase difference between the acceleration voltage signal $V_{acc}$ and the control radiofrequency power becomes within the predetermined value, the low power radiofrequency control system 25 inputs, to the accelerating cavity 9, as radiofrequency power, the acceleration radiofrequency power synchronized with the acceleration frequency that is the frequency of the acceleration voltage signal $V_{acc}$ by feedback control, and thereafter, when the acceleration frequency reaches the output frequency $F_{ext}$, stops the input of the acceleration radiofrequency power to the accelerating cavity 9. Thus it becomes possible to appropriately perform input and stop of the acceleration radiofrequency power according to the actual resonance pattern of the accelerating cavity 9, and therefore it becomes possible to appropriately accelerate the beam to desired energy. Therefore, it becomes possible to accurately extract a beam having desired energy.

In the present embodiment, the center of the orbit where the beam orbits is shifted from the center of the acceleration region 101. Therefore, it becomes possible to reduce the kick amount required for extraction of the beam, and it becomes possible to easily extract the beam having desired energy.

In the present embodiment, the beam is injected into the acceleration region 101 after a certain period of time from when the acceleration voltage value reaches the threshold value $V_{thrd}$. Alternatively, when the frequency becomes a predetermined frequency after the acceleration voltage value reaches the threshold value $V_{thrd}$, the beam is injected into the acceleration region 101. Therefore, it becomes possible to cause the beam to be injected at an appropriate timing, and it becomes possible to accurately extract a beam having desired energy.

In the present embodiment, the low power radiofrequency control system 25 generates acceleration radiofrequency power by, using the PLL circuit, subjecting an input measurement signal in which the radiofrequency power input to the accelerating cavity 9 is measured to phase synchronization with the acceleration voltage signal $V_{acc}$. In particular, in the PLL circuit, all circuits include digital circuits, and IQ modulation is used. Therefore, it becomes possible to easily perform input and stop of acceleration radiofrequency power.

In the present embodiment, since input and stop of the acceleration radiofrequency power are controlled according to the actual resonance pattern of the accelerating cavity 9, it becomes possible to appropriately accelerate the beam to desired energy even if the acceleration radiofrequency power is amplified using a semiconductor amplifier that takes a certain time to raise.

The above-described embodiment of the present disclosure is an example for describing the present disclosure, and is not intended to limit the scope of the present disclosure only to the embodiment. Those skilled in the art can practice the present disclosure in various other aspects without departing from the scope of the present disclosure.

Reference Signs List

1    oscillator
2    changeover switch

| 3 | TDC |
|---|---|
| 4 | digital loop filter |
| 5 | DCO |
| 6 | RF switch |
| 7 | radiofrequency power amplifier |
| 8 | input radiofrequency measurement circuit |
| 9 | accelerating cavity |
| 10 | acceleration voltage measurement circuit |
| 11 | acceleration gap |
| 12 | dee electrode |
| 13 | dummy dee electrode |
| 14 | inner conductor |
| 15 | outer conductor |
| 16 | voltage amplitude comparator |
| 17 | frequency comparator |
| 18 | controller |
| 19 | host control system |
| 20 | input coupler |
| 21 | pickup portion |
| 25 | low power radiofrequency control system |
| 30 | rotating capacitor |
| 31 | motor |
| 40 | main electromagnet |
| 41 | yoke |
| 42 | main coil |
| 43 | main magnetic pole |
| 50 | cryostat |
| 51 | ion source |
| 52 | low energy beam transport system |
| 53 | ion injection portion |
| 80 | high energy beam transport system |
| 81 | magnetic channel |
| 82 | entrance of beam extraction path |
| 90 | radiofrequency kicker |
| 91 | peeler magnetic field region |
| 92 | regenerator magnetic field region |
| 100 | accelerator |
| 101 | acceleration region |
| 500 | particle therapy system |
| 501 | treatment couch |
| 502 | beam transport device |
| 503 | irradiation device |

## Claims

1. An accelerator that accelerates a charged particle beam while causing the charged particle beam to orbit by a temporally constant main magnetic field and an acceleration radiofrequency voltage, the accelerator comprising:

   an accelerating cavity that excites the acceleration radiofrequency voltage according to a time-varying resonance frequency by using radiofrequency power having been input;
   a measurement unit that outputs a measurement signal obtained by measuring the acceleration radiofrequency voltage; and
   a control unit that inputs, as the radiofrequency power, control radiofrequency power having a constant frequency included in a range of the resonance frequency to the accelerating cavity, inputs, as the radiofrequency power, acceleration radiofrequency power synchronized with an acceleration frequency that is a frequency of the measurement signal by feedback control to the accelerating cavity when a phase difference between the measurement signal and the control radiofrequency power falls within a predetermined value, and stops input of the acceleration radiofrequency power to the accelerating cavity when the acceleration frequency reaches a required value.

2. The accelerator according to claim 1, wherein a center of an orbit where the charged particle beam orbits is shifted from a center of an acceleration region where the main magnetic field is excited.

3. The accelerator according to claim 1, wherein after a certain period of time from when the phase difference becomes within a predetermined value, the charged particle beam is injected into an acceleration region where the main magnetic field is excited.

4. The accelerator according to claim 1, wherein when the frequency becomes a predetermined frequency after the phase difference becomes within the predetermined value, the charged particle beam is injected into an acceleration region where the main magnetic field is excited.

5. The accelerator according to claim 1, wherein the control unit generates the acceleration radiofrequency power by, using a phase locked loop circuit, subjecting an input measurement signal in which the radiofrequency power is measured to phase synchronization with the measurement signal.

6. The accelerator according to claim 5, wherein the phase locked loop circuit uses IQ modulation.

7. The accelerator according to claim 6, wherein in the phase locked loop circuit, all circuits include digital circuits.

8. The accelerator according to claim 1, further comprising a semiconductor amplifier that amplifies the radiofrequency power.

9. A particle therapy system comprising:

   the accelerator according to claim 1; and
   an irradiation device that irradiates an irradiation target person with a charged particle beam extracted from the accelerator.

10. A control method of an accelerator that includes an accelerating cavity that excites an acceleration radiofrequency voltage corresponding to a time-varying resonance frequency using radiofrequency power having been input, and accelerates a charged particle beam while causing the charged particle beam to orbit by a temporally constant main magnetic field and the acceleration radiofrequency voltage, the control method comprising:

    outputting a measurement signal in which the acceleration radiofrequency voltage is measured;
    inputting, to the accelerating cavity, as the radiofrequency power, control radiofrequency power having a constant frequency included in a range of the resonance frequency;
    when a phase difference between the measurement signal and the control radiofrequency power becomes within a predetermined value, inputting, to the accelerating cavity as the radiofrequency power, acceleration radiofrequency power synchronized with an acceleration frequency that is a frequency of the measurement signal by feedback control; and
    stopping input of the acceleration radiofrequency power to the accelerating cavity when the acceleration frequency reaches a required value.

# FIG. 1

100

51
52
40
25
20
30
9
74
72
71
80
D

# FIG. 2

100

73
91
72
82
81
92
71
80
A
90
11
13
53
61
62
O
12
21
b
101
10
25
7
74
8
20
30
31
14
15
9
42
41
b'

# FIG. 3

# FIG. 4

# FIG. 5

ANGLE SIGNAL

16  $V_{thrd}$

RF ON

17  $F_{ext}$

10    9    8    7

$V_{acc}$

RF
OFF

18

2

SWITCHING SIGNAL

$I_{out}$

INPUT

6    $f_{out}$

REFERENCE

ON/OFF SIGNAL

3    4    5

1  $f_{pre}$

ACCELERATION
END SIGNAL

BEAM REQUEST
SIGNAL

25

19

# FIG. 6

# FIG. 7

<div align="center">

**INTERNATIONAL SEARCH REPORT**

</div>

| International application No. |
|---|
| **PCT/JP2022/030802** |

| A. | CLASSIFICATION OF SUBJECT MATTER |
|---|---|

*H05H 13/02*(2006.01)i; *A61N 5/10*(2006.01)i
FI: H05H13/02; A61N5/10 H

According to International Patent Classification (IPC) or to both national classification and IPC

| B. | FIELDS SEARCHED |
|---|---|

Minimum documentation searched (classification system followed by classification symbols)

H05H3/00-15/00; A61N5/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2020-202015 A (HITACHI LTD) 17 December 2020 (2020-12-17)<br>entire text, all drawings | 1-10 |
| A | JP 2020-38797 A (HITACHI LTD) 12 March 2020 (2020-03-12)<br>entire text, all drawings | 1-10 |
| A | JP 2019-133745 A (HITACHI LTD) 08 August 2019 (2019-08-08)<br>entire text, all drawings | 1-10 |
| A | JP 2021-35467 A (HITACHI LTD) 04 March 2021 (2021-03-04)<br>paragraphs [0021]-[0168], fig. 1-3 | 1-10 |
| A | JP 2021-7645 A (HITACHI LTD) 28 January 2021 (2021-01-28)<br>paragraphs [0072]-[0086], fig. 9 | 1-10 |
| A | JP 2008-507826 A (STILL RIVER SYSTEMS INC) 13 March 2008 (2008-03-13)<br>abstract, fig. 1 | 1-10 |
| A | JP 2011-507151 A (STILL RIVER SYSTEMS INC) 03 March 2011 (2011-03-03)<br>abstract | 1-10 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **29 September 2022** | **11 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2022/030802**

| Patent document<br>cited in search report | | | Publication date<br>(day/month/year) | Patent family member(s) | | | Publication date<br>(day/month/year) |
|---|---|---|---|---|---|---|---|
| JP | 2020-202015 | A | 17 December 2020 | (Family: none) | | | |
| JP | 2020-38797 | A | 12 March 2020 | US<br>entire text, all drawings<br>WO | 2021/0196984<br><br>2020/049755 | A1<br><br>A1 | |
| JP | 2019-133745 | A | 08 August 2019 | US<br>entire text, all drawings<br>WO<br>CN | 2021/0195725<br><br>2019/146211<br>111279801 | A1<br><br>A1<br>A | |
| JP | 2021-35467 | A | 04 March 2021 | (Family: none) | | | |
| JP | 2021-7645 | A | 28 January 2021 | WO<br>paragraphs [0072]-[0086], fig.<br>9 | 2021/002043 | A1 | |
| JP | 2008-507826 | A | 13 March 2008 | US<br>abstract, fig. 1<br>US<br>US<br>US<br>WO<br>EP<br>EP<br>EP<br>CA<br>CN<br>CN | 2007/0001128<br><br>2008/0218102<br>2010/0045213<br>2013/0127375<br>2006/012467<br>2259664<br>3294045<br>3557956<br>2574122<br>101061759<br>102036461 | A1<br><br>A1<br>A1<br>A1<br>A2<br>A2<br>A1<br>A1<br>A1<br>A<br>A | |
| JP | 2011-507151 | A | 03 March 2011 | US<br>abstract<br>WO<br>EP<br>CA<br>CN<br>TW | 2009/0140671<br><br>2009/073480<br>2232962<br>2707075<br>101933406<br>200939908 | A1<br><br>A2<br>A<br>A1<br>A<br>A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100045213 A
- JP 2019133745 A